# EUROPEAN PATENT SPECIFICATION

(11) **EP 1 599 626 B1**
(45) Date of publication and mention of the grant of the patent: **15.09.2010**
(21) Application number: 04715920.7
(22) Date of filing: 01.03.2004
(51) Int. Cl.: D04H 1/00, D01F 9/00, A61L 9/014

(54) **NONWOVEN SUPPORT BASED ON ACTIVATED CARBON FIBRES, AND USE**
ALS TRÄGERMATERIAL GEEIGNETES VLIES AUF BASIS VON AKTIVKOHLEFASERN UND SEINE VERWENDUNG
SUPPORT NON TISSE A BASE DE FIBRES DE CARBONE ACTIVE ET UTILISATION DE CE SUPPORT

(30) Priority: 04.03.2003 FR 0302609
(43) Date of publication of application: 30.11.2005
(73) Proprietor: Ahlstrom Research and Services, 38780 Pont-Evêque (FR); Ahlstrom Corporation, 00180 Helsinki (FI)
(72) Inventor: ESCAFFRE, Pascale, La Cote Saint Andre 38260 (FR); CARTIER, Noël, Vienne 38200 (FR)
(74) Representative: Vuillermoz, Bruno
(86) International application number: PCT/FI2004/000110
(87) International publication number: WO 2004/079075

(56) References cited:
- EP-A- 0 311 364
- EP-A- 0 794 223
- WO-A-02/066085
- GB-A- 2 165 865
- US-A- 4 699 896
- US-A- 5 731 065
- US-A- 5 897 821
- US-B1- 6 524 508

## Description

The invention relates to a nonwoven support associating activated carbon fibres and chitosan fibres. It also relates to the use of this support as a dressing or as a filtering medium, especially for the filtration of liquid, solid or gaseous effluents. In an advantageous embodiment, the support of the invention is used due to its antimicrobial properties, especially in the field of food packaging. Another application can be that of shoe soles.

The chitosan is a deacetylated product of chitin. As for chitin, it is a polysaccharide which can be found in natural state in the cellular walls of fungi or in the shells of crabs, lobsters, shrimps or other crustaceans.

The chitin and the chitosan have been known for a very long time in the medical field, especially for their healing ability, but also in the field of filtration, and especially for the fixation of metallic ions contained in the liquid effluents.

Thus, the document US-A-3 903 268 describes a woven or nonwoven support including chitin obtained from cellular walls of the fungi or shellfish, or from chitin derivatives.

The document GB-A-2 165 865 describes nonwoven dressings associating chitin and chitosan. In practice, the cultivation of a fungi named "hyphae" is subjected to a treatment in an alkaline solution allowing dissolving the proteins contained in the cellular walls. Considering the low degree of the alkaline treatment, the obtained product contains a mixture of chitin and chitosan. In practice, the obtained material is present in the form of a fibre mat able to integrate carbon fibres used for reinforcing the mechanical properties of the support. No reference is made to the possible use of activated carbon fibres instead and in place of carbon fibres.

The document EP-A-291587 also describes dressing made from fibers consisting of a mixture of chitin and chitosan. As above, fibers are obtained from a natural fungus (mucore mucedo) treated with a solution of sodium hydroxide allowing the dissolution of the proteins contained in the outer layer of the cell walls and the exposure of chitin and chitosan. Therefore, the product which is obtained corresponds to an uncertain mixture of chitin, chitosan and residual cell walls.

The document JP-02-127596 describes a paper support impregnated with a chitin and chitosan solution. In practice, the solution is put on the surface of the support, and then the mixture of chitin and chitosan is precipitated by drying or by alkaline treatment.

The document EP-A-311 364 describes the use of activated carbon fibres in dressings, thus allowing absorbing the odours coming from exudates of the wounds. In practice, the layer based on activated carbon fibres is present in the form of a woven or a nonwoven and is incorporated into a protective envelope also in the form of a woven or a nonwoven. Nothing in this document shows the presence, in the layer, of an agent allowing binding the carbon fibres to each other. Consequently, it can be expected that the layer based on active carbon, when it is present in the form of a nonwoven, disintegrates into the envelope due to the absence of cohesion between the fibres, the size of which is very small i.e. 2 - 100 micrometers.

The document WO 02/066085 describes a dressing associating at least two separate layers, respectively, a first layer based on active wood coal (charcoal) and a second layer having an antimicrobial effect. In practice, the first layer is present in the form of a fabric containing the wood coal, in powder-form. The second layer can be a woven, a nonwoven or a knitted fabric, the antimicrobial effect being able to be obtained according to three different methods. According to a first method, the textile material is impregnated with the antimicrobial agent. According to a second method, the textile fibres already contain the antimicrobial agent before the manufacturing of the textile. In this case, if the textile yarns or filaments are present in the form of polymers, the antimicrobial agent is introduced when the polymerisation takes place. According to a third method, the textile is coated with a film containing an antimicrobial agent. In a particular embodiment, the antimicrobial agent is composed, in addition to metallic ions, of chitin or chitosan. No information is given concerning the proportions and the physical and chemical characteristics of the used chitosan. One of the inconveniences of a dressing of this type is that, in sum, it has a very low activity insofar as it contains a considerable proportion of inert fibres making up the first and the second layers.

In other words, the problem that the invention proposes to solve is how to provide an integrated support, the activity of which is optimal. Consequently, the invention proposes a support, for example, a nonwoven support based on activated carbon and chitosan, which is **characterised in that** all or part of the activated carbon and chitosan are present in the form of fibres.

The Applicant has noticed that, in a very surprising way, the presence of chitosan fibres allowed assuring the cohesion of the activated carbon fibres with each other, especially by bonding. Nothing implicated in the prior art that the chitosan fibres would be able to bind the activated carbon fibres. According to the Applicant, this property could be the result of the manufacturing technique of the support by wet method e.g. on a paper machine. In fact, the hydration of the chitosan fibres would cause the formation of intermolecular hydrogen bonds allowing trapping the active carbon fibres into a chitosan fibre web. Further, another advantage of chitosan fibres is that they have this capacity to bind the activated carbon fibres without clogging the pores of the said activated carbon so that it preserves all its activity. When the support of the invention is used as a dressing, the function of the activated carbon fibres is to entrap physically the exudates, whereas the chitosan fibres act biologically on the healing, allowing the formation of a neo-tissue close to the healthy skin, and in the same way on disinfection due to their bacteriostatic and fungistatic character. When the support of the invention is used for the filtration of e.g. liquid effluents, the function of the activated carbon fibres is to entrap the molecules, especially the organic compounds or mineral salts, due to the porosity thereof, whereas the function of the chitosan fibres is to entrap the metals dissolved by chemical complexation of metallic ions with the free amine function of the chitosan. In other words and in all cases, the chitosan fibres do not only carry out the bonding function that could be performed by a conventional binding agent (however, to a lesser degree because such an agent would lead to clogging of the pores of the activated carbon) but also a biological or chemical function depending on the final application.

According to an essential characteristic of the invention, the support does not contain chitin as is the case in the known prior art documents of the Applicant, but instead and in place of the chitin exclusively chitosan in the form of fibres.

This specificity allows obtaining a pure product, perfectly characterized, and having a high deacetylation degree allowing "optimization of the desired properties".

In practice, the molecular weight of the chitosan making up the fibres is between 10⁴ and 10⁶ g.mol.l⁻¹, preferably between 10⁵ and 5 10⁵ g.mol, and the deacetylation degree over 80 %, advantageously over 95 %.

Moreover and according to another characteristic, the chitosan fibres have a length of between 2 and 50 mm, advantageously between of 5 and 15 mm and a diameter between of 0,1 and 50 micrometers, advantageously between 5 of 20 micrometers. In a particular embodiment, the chitosan fibres are nanofibres, that is to say chitosan fibres, the diameter of which is between 0,1 and 1 micrometers, advantageously about 0,5 micrometers. Generally, it corresponds to artificial fibers which means 100% chitosan.

According to the invention, all or part of the activated carbon or chitosan is present in the form of fibres. In practice, at least 50%, advantageously at least 70 %, by weight of the activated carbon is present in the form of fibres, the balance to 100 % consisting of powder and/or granules. Also, at least 50 %, advantageously at least 70 %, by weight of the chitosan is present in the form of fibres, the balance to 100 % consisting of a chitosan solution and/or dispersion and/or chitosan powder impregnation.

Advantageously, at least 50 %, preferably at least 70 %, by weight of the activated carbon is present in the form of fibres, the balance to 100 % consisting of powder and/or grains and at least 50%, preferably at least 70 %, by weight of the chitosan is present in the form of fibres, the balance to 100 % consisting of a chitosan solution and/or dispersion and/or a chitosan powder impregnation.

In a preferred embodiment, all of the activated carbon and chitosan is in the form of fibres.

According to another characteristic, the chitosan fibres are treated with a germicide able to make them microbicide. This embodiment is especially advantageous in the applications in which the microbicide effect is strived for. Without being exhaustive, the applications are the dressings. The shoe soles are also covered by the scope of the invention. Advantageously, the germicide is a metallic salt chosen from the group comprising the silver salt, the copper salt, the zinc salt and the platinum salt representing advantageously between 0, 01 and 2 %, preferably 1 %, by weight of chitosan fibres. In a first embodiment, the chitosan fibre is immersed into a metallic salt bath, drained and dried. In a second embodiment, the metallic salt solution is added during the manufacturing process of the support especially into the fibre suspension containing the chitosan fibres e.g. at pulper level.

The activated carbon fibres, also known by the expression "active carbon fibres" are fibres perfectly known to a person skilled in the art. Their manufacturing process and characteristics are more precisely described in the document FR 97.14704, incorporated herein by reference. It is clear that the carbon fibres can be manufactured with all kinds of methods known by the person skilled in the art.

Essentially, a first manufacturing process consists in subjecting a carbon fibre texture to an activation treatment. The carbon fibre texture is obtained directly from yarns or carbon fibres stemming from a carbon precursor by heat treatment, or from yarns or the carbon precursor fibres, the transformation heat treatment of the precursor being carried out after the forming of the texture.

A second process consists in impregnating a texture of carbon precursor fibres with a composition allowing, after charring, obtaining directly a texture activated in carbon fibres.

According to the invention, the activated carbon fibres have a length of between 3 and 50 mm and advantageously between 5 and 15 mm, and a diameter of between 1 and 25 microns and advantageously between 7 and 18 microns.

In an advantageous embodiment, a germicide is absorbed on the activated carbon fibres. In practice, the germicide is a metallic salt chosen from the group consisting of a silver salt, copper salt, zinc salt and platinum salt, representing advantageously between 0,01 and 1 %, preferably 0,05 %, by weight of the activated carbon fibres.

In a first embodiment, the support of the invention is present in the form of a monolayer structure comprising from 10 to 90 % by weight of the chitosan fibres and from 10 to 90 % by weight of the activated carbon fibres. The chitosan fibres allow bonding the active carbon fibres to each other, resulting in obtaining a satisfactory cohesion.

In an advantageous embodiment, the monolayer structure of the invention contains exclusively a mixture of chitosan fibres and activated carbon fibres. In this case, a 100 % active support is used, that is to say that none of the constituents remain inert in relation to the envisaged application. This is especially the case when the support is used as a dressing. In fact, in such a hypothesis, the chitosan acts upon the healing whereas the active carbon acts upon the exudates. In practice, the support contains at least 20 % by weight, advantageously at least 50 % by weight of the chitosan fibres, the balance to 100 % consisting of activated carbon fibres.

In the case in which the monolayer structure does not consist exclusively of "active" fibres (chitosan and activated carbon), it may contain even 30 % by weight of thermobonding fibres and/or inorganic and/or organic fibres. In any case, the mass of the monolayer structure is between 15 g/m² and 600 g/m², advantageously between 50 and 100 g/m².

In the rest of the description and in the claims, the term "thermobonding fibres" denotes fibres having a dimension between 1 and 30 mm, preferably in the order of 5mm, the average melting point of which being 60°C - 180°C, these fibres being able to melt during the manufacturing process of the support, so as to bind the fibres nearby, and allowing reinforcing the mechanical characteristics of the said support. In practice, the fibres are chosen so that they melt at a temperature at which the support is manufactured, which is about 100°C if the drying takes place by contact on a drying cylinder, and about 170°C if the support is dried e.g. in pulsed air furnaces.

The thermobonding fibres used in the invention can have one or two melting points, in the hypothesis in which the fibre is present in the form of a so-called "bicomponent" fibre corresponding to a fibre comprising two polymers having different physical and/or chemical characteristics, extruded from the same die in order to form only one filament. In practice, the thermobonding fibres used in the invention are PET- and PE-based bicomponent fibres.

In the same way, the expression "organic and/or inorganic fibres" denotes, among the organic fibres, especially the cellulose fibres, synthetic fibres e.g. of the polyester, polyethylene, polypropylene, polyamide and polyvinyl chloride type; the artificial fibres (e.g. viscose, cellulose acetate); the natural fibres (e.g. cotton, wool, wood pulp); and among the inorganic fibres, especially the mineral fibres (e.g. glass fibres, ceramic fibres).

In a second embodiment, the support of the invention is present in the form of a bilayer structure associating, respectively, a first layer comprising chitosan fibres, and a second layer comprising activated carbon fibres.

The layer based on chitosan fibres may have several structures. First of all, it can be made exclusively up of chitosan fibres, thus being 100 % active, especially vis-à-vis the healing. In another embodiment, the first layer contains also fibres chosen from the group comprising the thermobonding fibres of the same type as those described previously, the organic and/or inorganic fibres and the activated carbon fibres by themselves or as a mixture. In this case, the chitosan fibres represent at least 20 %, advantageously at least 50 % by weight of the layer. In all cases, the chitosan fibres present at the first layer/second layer interface allow binding at least part of the second layer carbon fibres.

The said second layer contains at least 20 %, advantageously at least 50 %, of the activated carbon fibres, the balance to 100 % consisting of fibres chosen from the group comprising the chitosan fibres, the thermobonding fibres, the organic and/or inorganic fibres, by themselves or as a mixture.

In an advantageous embodiment of the support, the balance to 100 % of the second layer consists exclusively of chitosan fibres. When the first layer and the second layer contain, respectively and exclusively, chitosan fibres and a mixture of activated carbon fibres and chitosan, the support obtained is 100 % active, that is to say none of the constituents remain inert with regard to the application in question. Further, the two faces are distinct and active and provide their own specific activity: on the one hand the healing, on the other hand the capturing of odours related to exudates.

According to another characteristic, the first layer based on chitosan fibres of the bilayer material has a mass of between 5 and 100 g/m², advantageously between 5 and 20 g/m², whereas the layer based on activated carbon fibres has a mass of between 20 and 600 g/m² and advantageously between 20 and 100 g/m².

In a third embodiment, the support of the invention is present in the form of a trilayer structure consisting of three successive layers, respectively of two external layers comprising chitosan fibres, and a medium layer comprising activated carbon fibres. In this embodiment, the mass of each external layer is between 5 and 100 g/m², advantageously between 5 and 20 g/m², whereas the mass of the medium layer is between 20 and 600 g/m².

In practice, the nature of the external layers corresponds to that of the first layer of the bilayer support. In other words, the external layers can be exclusively composed of chitosan fibres or of mixture associating chitosan fibres, thermobonding fibres, organic and/or inorganic fibres and activated carbon fibres, by themselves or as a mixture. According to the invention, the composition of the external layers can be different. However, and in order to facilitate the manufacturing process, the composition of the external layers is identical.

With regard to the intermediate layer, it contains in practice at least 20 %, advantageously at least 50 % by weight of the activated carbon fibres, the balance to 100 % consisting of organic and/or inorganic fibres, thermobonding fibres or chitosan fibres, by themselves or as a mixture.

In a fourth embodiment, the support, whether it is present in the form of a monolayer, bilayer or trilayer, is incorporated within a nonwoven envelope based on chitosan fibres. In practice, the chitosan fibres represent at least 20 %, advantageously 100 % by weight of the envelope. However, the envelope can also contain other fibres, especially organic and/or inorganic fibres.

In a fifth embodiment, the support, whether it is present in the form of a monolayer, bilayer or trilayer, is covered on both sides thereof of chitosan nanofibres e.g. by electrospinning technology. In this case the diameter of the fibres may drop to 0,1 microns, and more commonly to 0,5 micrometers.

In a sixth embodiment, the support contains 4 layers, respectively:
- a first layer consisting of 100 % of thermobonding fibres
- a second layer based on activated carbon, of which at least 50 %, advantageously 100 % in the form of fibres, and the composition of which corresponds to that of the medium layer of the trilayer support,
- a third layer consisting of 100 % of thermobonding fibres,
- a fourth layer consisting of chitosan nanofibres, deposited e.g. by electrospinnig technology.

In a seventh embodiment, the chitosan nanofibres are also settled on the first layer.

Whether the material of the invention is present in the monolayer, bilayer or trilayer form, it can be manufactured by a continuous wet method of production, especially on a wet method machine type. In some cases, the support can result of the combination of two or more layers, each obtained by dry laid and/or wet laid or other processes. Yet another option is to use a foam-laid process for producing the multilayered products.

As already said, the humidity creates H-bonds allowing binding the activated carbon fibres at the surface of the chitosan fibres.
In the case of bilayer or trilayer, at least one head box is provided on the machine for preparing the chitosan based fibre suspensions for the manufacturing of the external layers or the medium layer according to the case, the attachment of the two or three layers being obtained by drainage and then by dewatering.

As a result of the properties of the activated carbon, especially on the deodorizing, and those of the chitosan fibres on the attachment of the metallic ions, a first application of the material of the invention is used for the filtration of liquid or gaseous effluents.

Consequently, the invention covers also the use of the previously described supports for the filtration of gaseous or liquid effluents. To be more precise, the treatment method of liquid or gaseous effluents consists in putting the support of the invention in contact with the said effluent; by frontal or tangential filtration.

On the other hand and considering the healing properties of chitosan, the material of the invention can also be used in the medical field, especially as a dressing. In this case, the invention relates also to a healing process of a wound consisting in applying the previously described support on the said wound.

Further, the association of chitosan fibres and active carbon fibres allow improving considerably the regeneration process of the tissues.

The antimicrobial properties of the chitosan fibres, when they are treated with heavy metals, make the support of the invention a support usable as a shoe sole.

The invention and the advantages which stem therefrom will become more apparent from the following illustrative examples.

### Example 1 : monolayer support

A monolayer material of 100 g/m², containing 50 % by weight of the chitosan fibres sold by France Chitine and 50 % by weight of the activated carbon fibres sold by UNITIKA under the reference A-10, was prepared.

The support was manufactured on a paper machine, by wet method, by putting on the wire the fibre composition containing the two types of fibres prepared beforehand. The obtained support is then dried by dewatering.

### Example 2: bilayer support

A bilayer support was prepared with the following composition:
*First layer:*
   - 100 % of chitosan fibres sold by France Chitine
   - mass of the layer: 20 g/m²
*Second layer:*
   - activated carbon fibres sold by the name Unitika A10: 80 % by weight of the layer
   - thermobonding fibres sold by the name PET bicomposant N 720H (KURARAY): 20 % by weight of the layer
   - mass of the second layer: 30 g/m²

The support was manufactured on a paper machine, by wet method, by providing two head boxes on the machine, respectively, a first head box containing the chitosan fibre composition, and a second head box containing a mixture of active carbon fibres and thermobonding fibres, the attachment of the two layers being obtained by dewatering.

### Example 3: trilayer support

A support, the composition of which is the following, was prepared:
*External la yers:*
   - 100 % of chitosan fibres sold by France Chitine
   - mass of the external layers: 10 g/m²
*Medium layer:*
   - 70 % of activated carbon fibres sold by the name Unitika A10
   - 30 % of chitosan fibres identical with the external layers
   - mass of the medium layer: 50 g/m².

The support was manufactured on a paper machine, by wet method, by providing three head boxes, respectively, a first head box comprising the mixture of chitosan, a second head box comprising the mixture of activated carbon fibres, and a third head box comprising the mixture of chitosan fibres, the attachment of the three layers being obtained by dewatering.

### Example 4: support with 4 layers

*Layer 1:*
   - 100 % of thermobonding fibres N 720H (KURARAY): mass: 10 g/m²
*Layer 2:*
   - 80 % of activated carbon fibres sold under the name Unitika A10
   - 20 % of N 720H fibres (KURARAY): identical with those of the layer 1
   - mass of the medium layer: 50 g/m².
*Layer 3:*
   - 100 % thermobonding fibres N 720H (KURARAY): mass of the external layers: 10 g/m²
*Layer 4:*
   - 100 % of chitosan nanofibres deposited by electrospinning
   - mass of the layer 3 g/m²

The support is manufactured on a paper machine, by wet method, by providing three head boxes, respectively a first head box comprising the synthetic thermobonding fibres, a second head box comprising the mixture of activated carbon fibres, and a third head box comprising the mixture of synthetic thermobonding fibres, the attachment of the three layers being obtained by dewatering. The fourth layer of chitosan nanofibres is deposited to the surface of the trilayer by electrospinning.

## Claims

1. A support based on activated carbon and chitosan, which is **characterised in that** all or part of the activated carbon and chitosan is present in the form of fibres.

2. A support according to claim 1, **characterised in that** at least 50 %, advantageously at least 70 % by weight of the activated carbon is present in the form of fibres, the balance to 100 % consisting of powder and/or grains.

3. A support according to claim 1, **characterised in that** at least 50 %, advantageously at least 70 %, by weight of the chitosan is present in the form of fibres, the balance to 100 % consisting of a chitosan solution and/or dispersion and/or a chitosan powder impregnation.

4. A support according to claim 1, **characterised in that** at least 50 %, advantageously at least 70 %, by weight of the activated carbon is present in the form of fibres, the balance to 100 % consisting of powder and/or grains and at least 50 %, advantageously at least 70 %, by weight of the chitosan is present in the form of fibres, the balance to 100 % consisting of a chitosan solution and/or dispersion and/or chitosan powder impregnation.

5. A support according to claim 1, **characterised in that** all of the activated carbon and the chitosan is present in the form of fibres.

6. A support according to claim 1, **characterised in that** the molecular weight of the chitosan making up the fibres is between 10⁴ and 10⁶ g.mol.l⁻¹, preferably between 10⁵ and 5 10⁵ g.mol./l, and the deacetylation degree over 80 %, advantageously over 95 %.

7. A support according to claim 1, **characterised in that** the chitosan fibres have a length of between 2 and 50 mm, advantageously between 5 and 15 mm and a diameter of between 0,1 and 50 microns.

8. A support according to claim 1, **characterised in that** the activated carbon fibres have a length of between 3 and 50 mm, advantageously between 5 and 15 mm and a diameter of between 1 and 25 microns, advantageously between 7 and 18 microns.

9. A support according to claim 1, **characterised in that** the germicide is absorbed on the activated carbon fibres and/or the chitosan fibres.

10. A support according to claim 9, **characterised in that** the germicide is a metallic salt chosen from the group comprising the silver salt, the copper salt, the zinc salt and the platinum salt.

11. A support according to claim 1, **characterised in that** it is present in the form of a monolayer structure comprising from 10 to 90 % by weight of chitosan fibres and from 10 to 90 % by weight of activated carbon fibres.

12. A support according to claim 11, **characterised in that** the monolayer structure consists exclusively of the activated carbon fibres and chitosan fibres.

13. A support according to claim 11, **characterised in that** the support consists at least 20 % by weight, advantageously at least 50 % by weight of chitosan fibres, the balance to 100 % consisting of activated carbon fibres.

14. A support according to claim 1, **characterised in that** it is present in the form of a bilayer structure associating, respectively, a first layer comprising chitosan fibres, and a second layer comprising activated carbon fibres.

15. A support according to claim 14, **characterised in that** the first layer contains exclusively chitosan fibres.

16. A support according to claim 14, **characterised in that** the first layer consists at least 20 %, advantageously at least 50 %, by weight of the layer of chitosan fibres, the balance to 100 % consisting of fibres chosen from the group comprising the thermobonding fibres, the organic and/or inorganic fibres and the activated carbon fibres, by themselves or as a mixture.

17. A support according to claim 14, **characterised in that** the second layer consists at least 20 % by weight, advantageously at least 50 % by weight of activated carbon fibres, the balance to 100 % consisting of fibres chosen from the group comprising the chitosan fibres, the thermobonding fibres, the organic and/or inorganic fibres, by themselves or as a mixture.

18. A support according to claim 17, **characterised in that** the balance to 100 % consists exclusively of chitosan fibres.

19. A support according to claim 1, **characterised in that** it is present in the form of a trilayer structure consisting of three successive layers, respectively of two external layers comprising chitosan fibres, and a medium layer comprising activated carbon fibres.

20. A support according to claim 19, **characterised in that** the external layers contain exclusively chitosan fibres.

21. A support according to claim 19, **characterised in that** the external layers consist at least 20 %, advantageously 50 %, by weight of the layer of chitosan fibres, the balance to 100 % consisting of fibres chosen from the group comprising the thermobonding fibres, the organic and/or inorganic fibres and the activated carbon fibres, by themselves or as a mixture.

22. A support according to claim 19, **characterised in that** the intermediate layer consists at least 20 %, advantageously at least 50 %, by weight of the activated carbon fibres, the balance to 100 % consisting of the organic and/or inorganic fibres, the thermobonding fibres or the chitosan fibres, by themselves or as a mixture.

23. A support according to claim 1, **characterised in that** it is incorporated within a nonwoven envelope containing chitosan fibres.

24. A support according to claim 23, **characterised in that** the chitosan fibres represent at least 20 %, advantageously 100 % by weight of the envelope.

25. A support according to claim 1, **characterised in that** it is covered from both sides thereof with chitosan nanofibres.

26. A support according to claim 1, **characterised in that** it is manufactured by continuous wet method on a machine of the wet method type.

27. Use of the support object of the claim 1, as a dressing.

## Patentansprüche

1. Träger oder Schicht für einen Mehrschichtträger in Form eines nichtgewebten textilen Flächengebildes, der auf der Verwendung von Aktivkohle und Chitosan beruht, **dadurch gekennzeichnet, dass** die Aktivkohle und das Chitosan im Ganzen oder zum Teil in Form von Fasern vorhanden sind, und dass die Chitosanfasern zum Verbinden der Aktivkohlefasern verwendet werden, um den Träger oder die Schicht für einen Mehrschichtträger zu bilden.

2. Träger oder Schicht für einen Mehrschichtträger nach Anspruch 1, **dadurch gekennzeichnet, dass** mindestens 50 Gew.-%, vorteilhafter Weise mindestens 70 Gew.-% der Aktivkohle in Form von Fasern vorhanden sind, wobei der auf 100% fehlende Rest aus Pulver und/oder Körnchen besteht.

3. Träger oder Schicht für einen Mehrschichtträger nach Anspruch 1, **dadurch gekennzeichnet, dass** mindestens 50 Gew.-%, vorteilhafter Weise mindestens 70 Gew.% des Chitosans in Form von Fasern vorhanden sind, wobei der auf 100% fehlende Rest aus einer Chitosanlösung und/oder -dispersion und/oder einer Chitosanpulver-Tränkung besteht.

4. Träger oder Schicht für einen Mehrschichtträger nach Anspruch 1, **dadurch gekennzeichnet, dass** mindestens 50 Gew.-%, vorteilhafter Weise mindestens 70 Gew.-% der Aktivkohle in Form von Fasern vorhanden sind, wobei der auf 100% fehlende Rest aus Pulver und/oder Körnchen besteht, und mindestens 50 Gew.-%, vorteilhafter Weise mindestens 70 Gew.% des Chitosans in Form von Fasern vorhanden sind, wobei der auf 100% fehlende Rest aus einer Chitosanlösung und/oder -dispersion und/oder einer Chitosanpulver-Tränkung besteht.

5. Träger oder Schicht für einen Mehrschichtträger nach Anspruch 1, **dadurch gekennzeichnet, dass** die Aktivkohle und das Chitosan in Gänze in Form von Fasern vorhanden sind.

6. Träger oder Schicht für einen Mehrschichtträger nach Anspruch 1, **dadurch gekennzeichnet, dass** das Molekulargewicht des Chitosans, das die Fasern ausmacht, zwischen 10⁴ und 10⁶ g.mol.l⁻¹, vorzugsweise zwischen 10⁵ und 5*10⁵ g.mol.l beträgt, und der Deacetylierungsgrad über 80%, vorteilhafter Weise über 95% ist.

7. Träger oder Schicht für einen Mehrschichtträger nach Anspruch 1, **dadurch gekennzeichnet, dass** die Chitosanfasern eine Länge zwischen 2 und 50 mm, vorteilhafter Weise zwischen 5 und 15 mm, und einen Durchmesser zwischen 0,1 und 50 Mikrometer haben.

8. Träger oder Schicht für einen Mehrschichtträger nach Anspruch 1, **dadurch gekennzeichnet, dass** die Aktivkohlefasern eine Länge zwischen 3 und 50 mm, vorteilhafter Weise zwischen 5 und 15 mm, und einen Durchmesser zwischen 1 und 25 Mikrometer, vorteilhafter Weise zwischen 7 und 18 Mikrometer haben.

9. Träger oder Schicht für einen Mehrschichtträger nach Anspruch 1, **dadurch gekennzeichnet, dass** ein Desinfektionsmittel in den Aktivkohlefasern und/oder Chitosanfasern absorbiert ist.

10. Träger oder Schicht für einen Mehrschichtträger nach Anspruch 9, **dadurch gekennzeichnet, dass** das Desinfektionsmittel ein Metallsalz ist, das aus der Gruppe ausgewählt ist, die Silber-, Kupfer-, Zink- und Platinsalz umfasst.

11. Träger nach Anspruch 1, **dadurch gekennzeichnet, dass** der Träger in Form einer Einschichtstruktur vorliegt, die 10 bis 90 Gew.-% Chitosanfasern und 10 bis 90 Gew.-% Aktivkohlefasern umfasst.

12. Träger nach Anspruch 11, **dadurch gekennzeichnet, dass** die Einschichtstruktur ausschließlich aus den Aktivkohle- und Chitosanfasern besteht.

13. Träger nach Anspruch 11, **dadurch gekennzeichnet, dass** der Träger zu mindestens 20 Gew.-%, vorteilhafter Weise mindestens 50 Gew.-% aus Chitosanfasern besteht, wobei der auf 100% fehlende Rest aus Aktivkohlefasern besteht.

14. Träger nach Anspruch 1, **dadurch gekennzeichnet, dass** der Träger in Form einer Zweischichtstruktur vorliegt, der eine erste Schicht, die zumindest Chitosanfasern umfasst, und eine zweite Schicht, die zumindest Aktivkohlefasern umfasst, zugeordnet ist.

15. Träger nach Anspruch 14, **dadurch gekennzeichnet, dass** die erste Schicht ausschließlich Chitosanfasern enthält.

16. Träger nach Anspruch 14, **dadurch gekennzeichnet, dass** die erste Schicht zu mindestens 20 Gew.-%, vorteilhafter Weise mindestens 50 Gew.-% der Schicht aus Chitosanfasern besteht, wobei der auf 100% fehlende Rest aus wärmeverbindbaren Fasern, organischen Fasern, anorganischen Fasern und/oder Aktivkohlefasern besteht.

17. Träger nach Anspruch 14, **dadurch gekennzeichnet, dass** die zweite Schicht zu mindestens 20 Gew.-%, vorteilhafter Weise mindestens 50 Gew.% aus Aktivkohlefasern besteht, wobei der auf 100% fehlende Rest aus Chitosanfasern, wärmeverbindenden Fasern, organischen Fasern und/oder anorganischen Fasern besteht.

18. Träger nach Anspruch 17, **dadurch gekennzeichnet, dass** der auf 100% fehlende Rest ausschließlich aus Chitosanfasern besteht.

19. Träger nach Anspruch 1, **dadurch gekennzeichnet, dass** der Träger in Form einer Dreischichtstruktur vorliegt, die aus drei aufeinanderfolgenden Schichten besteht, und zwar jeweils zwei äußeren Schichten, die zumindest Chitosanfasern umfassen, und einer Zwischenschicht, die zumindest Aktivkohlefasern umfasst.

20. Träger nach Anspruch 19, **dadurch gekennzeichnet, dass** die äußeren Schichten ausschließlich Chitosanfasern enthalten.

21. Träger nach Anspruch 19, **dadurch gekennzeichnet, dass** die äußeren Schichten zu mindestens 20 Gew.-%, vorteilhafter Weise 50 Gew.-% der Schicht aus Chitosanfasern bestehen, wobei der auf 100% fehlende Rest aus wärmeverbindenden Fasern, organischen Fasern, anorganischen Fasern und/oder Aktivkohlefasern besteht.

22. Träger nach Anspruch 19, **dadurch gekennzeichnet, dass** die Zwischenschicht zu mindestens 20 Gew.-%, vorteilhafter Weise mindestens 50 Gew.-% aus Aktivkohlefasern besteht, wobei der auf 100% fehlende Rest aus organischen Fasern, anorganischen Fasern, wärmeverbindenden Fasern und/oder Chitosanfasern besteht.

23. Träger oder Schicht für einen Mehrschichtträger nach Anspruch 23, **dadurch gekennzeichnet, dass** er bzw. sie in eine nichtgewebte Hülle eingeschlossen ist, die Chitosanfasern enthält.

24. Träger oder Schicht für einen Mehrschichtträger nach Anspruch 1, **dadurch gekennzeichnet, dass** die Chitosanfasern mindestens 20 Gew.-%, vorteilhafter Weise 100 Gew.-% der Hülle darstellen.

25. Träger oder Schicht für einen Mehrschichtträger nach Anspruch 1, **dadurch gekennzeichnet, dass** er bzw. sie von seinen/ihren beiden Seiten her mit Chitosan-Nanofasern bedeckt ist.

26. Träger oder Schicht für einen Mehrschichtträger nach Anspruch 1, **dadurch gekennzeichnet, dass** er bzw. sie durch ein kontinuierlichen Nassverfahren auf einer Maschine der Nassverfahrensbauart, d.h. mittels eines sogenannten Papiermaschinenprozesses hergestellt wird.

27. Verwendung des erfindungsgegenständlichen Trägers nach Anspruch 1 als Wundverband.

## Revendications

1. Support ou couche pour support multi couches sous forme de non tissé, à base de carbone activé et de chitosane, qui se **caractérise en ce que** tout ou partie du carbone activé et du chitosane se présentent sous la forme de fibres et **en ce que** les fibres de chitosane ont pour fonction de lier les fibres de carbone activé pour former le support ou ladite couche pour support multi-couches.

2. Support ou couche pour support multi couches selon la revendication 1, **caractérisé en ce que** au moins 50%, avantageusement au moins 70% en poids du carbone activé se présente sous la forme de fibres, le complément à 100% étant constitué de poudre et/ou de granules.

3. Support ou couche pour support multi couches selon la revendication 1, **caractérisé en ce que** au moins 50%, avantageusement au moins 70% en poids du chitosane se présente sous la forme de fibres, le complément à 100% étant constitué constitué d'une imprégnation d'une solution et/ou d'une dispersion de chitosane et/ou d'une poudre de chitosane.

4. Support ou couche pour support multi couches selon la revendication 1, **caractérisé en ce que** au moins 50%, avantageusement au moins 70% en poids du carbone activé se présente sous la forme de fibres, le complément à 100% étant constitué de poudre et/ou de granules et au moins 50%, avantageusement au moins 70% en poids du chitosane se présente sous la forme de fibres, le complément à 100% étant constitué d'une imprégnation d'une solution et/ou d'une dispersion de chitosane et/ou d'une poudre de chitosane.

5. Support ou couche pour support multi couches selon la revendication 1, **caractérisé en ce que** tout le carbone activé et le chitosane se présentent sous forme de fibres.

6. Support ou couche pour support multi couches selon la revendication 1, **caractérisé en ce que** le poids moléculaire du chitosane constitutif des fibres est compris entre 10⁴ et 10⁶ g.mol.l⁻¹, de préférence compris entre 10⁵ et 5 10⁵ g.mol./l, et le degré de désacétylation supérieur à 80 %, avantageusement supérieur à 95 %.

7. Support ou couche pour support multi couches selon la revendication 1, **caractérisé en ce que** les fibres de chitosane ont une longueur comprise entre 2 et 50 mm, avantageusement entre 5 et 15 mm et un diamètre compris entre 0,1 et 50 microns.

8. Support ou couche pour support multi couches selon la revendication 1, **caractérisé en ce que** les fibres de carbone activé ont une longueur comprise entre 3 et 50 mm, avantageusement entre 5 et 15 mm et un diamètre compris entre 1 et 25 microns avantageusement entre 7 et 18 microns

9. Support ou couche pour support multi couches selon la revendication 1, **caractérisé en ce qu'**un agent germicide est adsorbé sur les fibres de carbone activé et/ou les fibres de chitosane.

10. Support ou couche pour support multi couches selon la revendication 9, **caractérisé en ce que** l'agent germicide est un sel métallique choisi dans le groupe comprenant le sel d'argent, de cuivre, de zinc, de platine.

11. Support selon la revendication 1, **caractérisé en ce qu'**il se présente sous forme d'une structure monocouche comprenant de 10 à 90 % en poids de fibres de chitosane et de 10 à 90 % en poids de fibres de carbone activé.

12. Support selon la revendication 11, **caractérisé en ce que** la structure monocouche contient exclusivement des fibres de carbone activé et des fibres de chitosane.

13. Support selon la revendication 11, **caractérisé en ce** le support contient au moins 20 % en poids, avantageusement au moins 50 % en poids de fibres de chitosane, le complément à 100 % étant constitué de fibres de carbone activé.

14. Support selon la revendication 1, **caractérisé en ce qu'**il se présente sous forme d'une structure bicouche associant respectivement une première couche comprenant des fibres de chitosane, et une seconde couche comprenant des fibres de carbone activé.

15. Support selon la revendication 14, **caractérisé en ce que** la première couche contient exclusivement des fibres de chitosane.

16. Support selon la revendication 14, **caractérisé en ce que** la première couche contient au moins 20 %, avantageusement au moins 50 % en poids de la couche, de fibres de chitosane, le complément à 100% étant constitué par au moins une des fibres choisies parmi les fibres thermoliantes, les fibres organiques et/ou inorganiques et les fibres de carbone activé.

17. Support selon la revendication 14, **caractérisé en ce que** la seconde couche contient au moins 20% en poids, avantageusement au moins 50% en poids de fibres de carbone activé, le complément à 100 % étant constitué par au moins une des fibres choisies parmi les fibres de chitosane, les fibres thermoliantes, les fibres organiques et/ou inorganiques.

18. Support selon la revendication 17, **caractérisé en ce que** le complément à 100 % est constitué exclusivement de fibres de chitosane.

19. Support selon la revendication 1, **caractérisé en ce qu'**il se présente sous forme d'une structure tricouche constituée de trois couches successives, respectivement deux couches externes comprenant des fibres de chitosane, et une couche médiane comprenant des fibres de carbone activé.

20. Support selon la revendication 19, **caractérisé en ce que** les couches externes contiennent exclusivement des fibres de chitosane.

21. Support selon la revendication 19, **caractérisé en ce que** les couches externes contiennent au moins 20 %, avantageusement 50 % en poids de la couche, de fibres de chitosane, le complément à 100% étant constitué par au moins une des fibres choisies parmi les fibres thermoliantes, les fibres organiques et/ou inorganiques et les fibres de carbone activé.

22. Support selon la revendication 19, **caractérisé en ce que** la couche intermédiaire contient au minimum 20 %, avantageusement au moins 50 % en poids de fibres de carbone activé, le complément à 100 % étant constitué par au moins une des fibres choisies parmi les fibres organiques et/ou inorganiques, les fibres thermoliantes ou les fibres de chitosane.

23. Support ou couche pour support multi couches selon la revendication 1, **caractérisé en ce qu'**il est incorporé au sein d'une enveloppe non tissée contenant des fibres de chitosane.

24. Support ou couche pour support multi couches selon la revendication 23, **caractérisé en ce que** les fibres de chitosane représentent au moins 20% avantageusement 100 % en poids de l'enveloppe.

25. Support ou couche pour support multi couches selon la revendication 1, **caractérisé en ce qu'**il est recouvert, de part et d'autre de nanofibres de chitosane.

26. Support ou couche pour support multi couches selon la revendication 1, **caractérisé en ce qu'**il est fabriqué par voie humide en continu, sur une machine type voie humide, c'est-à-dire au moyen d'une procédé par voie papetière.

27. Utilisation du support objet de la revendication 1, comme pansement.
